# EUROPEAN PATENT APPLICATION

(11) **EP 3 111 910 A1**
(43) Date of publication of application: **04.01.2017**
(21) Application number: 15755764.6
(22) Date of filing: 05.02.2015
(51) Int. Cl.: A61J 1/10, G09F 3/00

(54) **SECURITY DEVICE APPLICABLE TO CONTAINERS CONTAINING PARENTERAL THERAPIES**

(30) Priority: 27.02.2014 ES 201430263 U
(71) Applicant: Jorge Urtiaga Baonza Solutions, S.L., 08402 Barcelona (ES)
(72) Inventor: URTIAGA BAONZA, Jorge, 08402 Barcelona (ES)
(74) Representative: Gallego Jiménez, José Fernando
(86) International application number: PCT/ES2015/070077
(87) International publication number: WO 2015/128521

(57) **Abstract**

The invention relates to a security device applicable to containers containing parenteral therapies, such as medicaments, blood products or parenteral nutrition, especially containers (E) provided with an eyelet (O) for hanging the container upside down during administration of the parenteral therapy by gravity. The device comprises: a body having a first part (1) and a second part (2) respectively provided with facing surfaces (11, 21) designed in terms of the shape and dimensions thereof so as to cover the eyelet (O) of the container (E) and render it unusable; and a closing mechanism (3) which immobilizes the device in relation to the container (E) in a position wherein said device covers the eyelet (O), rendering it temporarily unusable.

## Description

### Object of the invention

The present invention relates to a safety device applicable to containers containing parenteral therapies, such as drugs, blood products or parenteral nutrition; and in particular to those containers provided with an eyelet for hanging in an inverted position during gravity flow administration of the parenteral therapy to a patient.

### Technical field of the invention

This device is applicable in the field of the manufacture of safety elements applicable in all types of hospitals, clinics, health centres and home care in administering by flow gravity of therapies to patients, particularly blood and drugs transfusions, from a container containing the product in question.

### Background of the invention

The administration of parenteral therapies to patients (whether in hospitals, health centres or sometimes in the patient's own home) requires a safe and strict control and identification system to ensure the security in the administration of such therapies and avoid errors which can lead to significant adverse effects on patients.

These systems are mainly based on computer systems that identify the patient and the product to be administered and inform the person responsible for the administration of such therapy if the patient and the product to be administered are correct or not.

There are other devices, as that described in the utility model ES1071023 U, which are based on the use of a container having an inner cavity to safely house the container containing the product or therapy to be given to a specific patient, said container having a lid provided with a locking mechanism that requires the identification of the patient for which it is intended in order to be opened.

This device is particularly suitable to control the container containing the product or therapy to be given during its distribution and transport, but it poses logistical problems, since it is based on the use of a vessel larger than the container itself containing the product or therapy to be given. Another drawback of this device in that, in the event of any error or system failure that prevents the opening of the outer container, it is impossible to access the interior container and give the therapy to the patient, which may be vital in medical emergencies.

The technical problem that arises is the development of a safety device applicable to containers containing parenteral therapies that provides physical safety associated with the logical security which currently exists in many hospitals or health centres, significantly increasing security; keeping the container accessible without keep it confined in a container, and allowing access to the container and the administration of the treatment to the patient in an emergency.

### Description of the invention

The safety device for the administration of parenteral products object of this invention has technical particularities intended to ensure the correct administration of parenteral products to a patient for which they were prescribed and subsequently prepared at different hospital services (pharmacy, blood bank, bone marrow bank, etc.).

The device is applicable in those containers which have an eyelet for hanging from a pole or support during the administration of the product to the patient by gravity flow.

According to the invention, the device comprises: a body having a first portion and a second portion provided with respective facing surfaces adapted in size and shape to cover and render the eyelet of the container unusable; and a locking mechanism which locks the device relative to the container in a position in which said device covers the eyelet of the container by making it temporarily unusable.

With the above features the device allows for blocking the use of the eyelet existing on the containers for products or parenteral treatments (blood or drugs), preventing such containers from being hung for administration of its content to the patient by gravity flow.

The locking mechanism can have any kind of actuating mechanism as this does not affect the essence of the invention.

It is envisaged that said locking mechanism can be mechanically actuated, for example by a key; or be actuated electronically, with the first portion of the device body containing all the electronic and communication systems, batteries and mechanical systems necessary to lock or unlock the pin by sending, by wire or wireless, a code or combination in accordance to the security protocols of each administration centre.

The device body may be comprised of two separate parts that make up the first portion and the second portion thereof; although it is possible that said device body can be a single piece, with the first and second portion thereof being separated from a portion of the container provided with the eyelet to be locked by an insertion slot between their respective facing surfaces.

### Description of the figures

In order to complement the description that is being carried out and with the purpose of facilitating the understanding of the characteristics of the invention, the present description is accompanied by a set of drawings wherein, by way of a non-limiting example, the following has been represented:

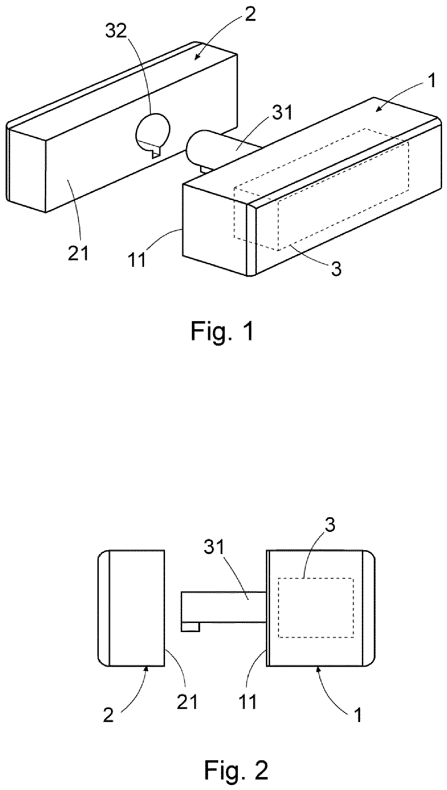

- Figure 1 shows a schematic perspective view of an embodiment of the safety device applicable to containers containing parenteral therapies according to the invention, consisting of two independent or separable parts.
- Figure 2 shows a profile view of the device of Figure 1 with the two parts separated.
- Figure 3 shows an exploded perspective view of an embodiment of the safety device according to the invention, wherein the body thereof comprises two independent parts which are shown separated and facing the eyelet of a container containing a parenteral therapy.
- Figure 4 shows a view analogous to the above with the device in an operational use position, fully covering the eyelet of the bag.
- Figure 5 corresponds to a profile view of Figure 4

### Preferred embodiment of the invention

In the embodiment shown in the accompanying figures the device comprises a body with a first portion (1) and a second portion (2) constituted in this case by two independent pieces having respective surfaces (11, 21) facing each other.

In the first portion (1) of the device body a locking mechanism (3) is mounted, which in this case is electronically operated wirelessly and is provided with a rotatory pin (31).

The second portion (2) of the device body has a cavity (32) for the introduction and fixing of the locking pin (31).

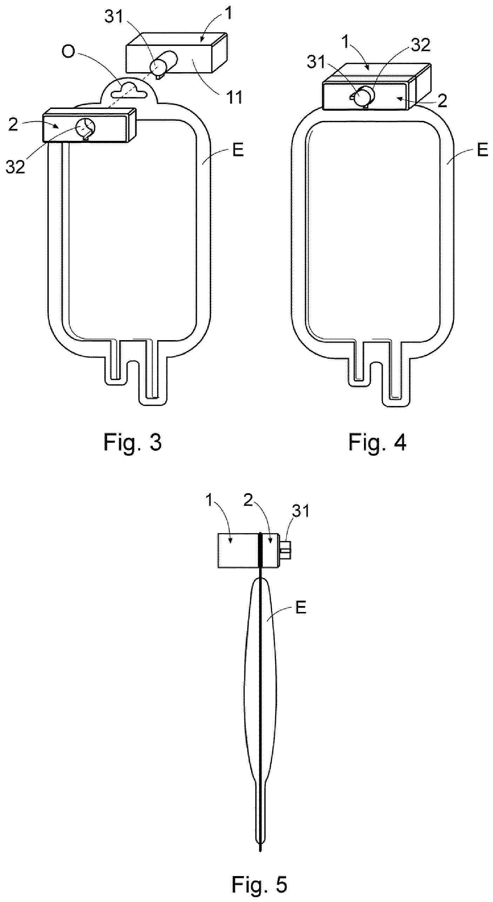

As shown in Figure 3 the pin (31) of the locking mechanism is intended to be inserted through the eyelet "O" of a container "E" containing the parental therapy to be given by gravity flow to a patient and introduced through the cavity (32) of the second portion (2) of the device body, the fixation of the first portion and the second portion of the device body being established through the rotation of the pin (31), so that the facing surfaces (11, 21) of the first portion (1) and the second portion (2) completely cover the area of the container (E) carrying the eyelet (O) as shown in Figure 4 and 5.

In this position of use of the device, the eyelet (O) is blocked, thus preventing it from being used to hang the container (E) in a pole or similar support. However, this does not prevent the container (E) from being hand-held to deliver the product contained therein to a patient in an emergency.

## Claims

1. Safety device applicable to containers containing parenteral therapies such as drugs, blood products or parenteral nutrition; and in particular to those containers (E) provided with an eyelet (O) for hanging them in an inverted position during administration by gravity flow of the parenteral therapy; comprising a body having a first portion (1) and a second portion (2) provided with respective facing surfaces (11, 21) adapted in size and shape to cover and render the eyelet (O) of the container (E) unusable; and a locking mechanism (3) which locks the device relative to the container (E) in a position in which said device covers the eyelet (O) by making it temporarily unusable **characterised in that** the locking mechanism (3) is mechanically actuated, by a key; or electronically actuated and is provided with a rotatory pin (1); the first portion (1) of the device containing all electronic and communication systems, batteries and mechanical systems necessary to lock or unlock the pin by sending, by wire or wireless, a code or combination in accordance to the security protocols of each administration centre; the second portion (2) of the body comprising a cavity (32) for the introduction and fixing of the pin (31) in a locked position.

2. Safety device according to claim 1, **characterised in that** the device body consists of two independent parts forming the first portion (1) and the second portion (2) thereof.

3. Safety device according to claim 1, **characterised in that** the device body is a single piece, the first portion (1) and the second portion (2) thereof being separated by a slot for introducing a portion of the container (E) provided with the eyelet (O) to be blocked.
